Europäisches Patentamt

European Patent Office

Office européen des brevets

(11) Publication number: **0 464 140 B1**

EUROPEAN PATENT SPECIFICATION

(45) Date of publication of patent specification: **27.07.94**    (51) Int. Cl.⁵: **C07K  7/10, C07K  17/02,**
**C07K  17/06, C07K  17/14**

(21) Application number: **90905829.9**

(22) Date of filing: **20.03.90**

(86) International application number:
**PCT/US90/01486**

(87) International publication number:
**WO 90/11297 (04.10.90 90/23)**

The file contains technical information submitted
after the application was filed and not included in
this specification

(54) **HYDROPHOBIC ATTACHMENT SITE FOR ADHESION PEPTIDES.**

(30) Priority: **20.03.89 US 326168**

(43) Date of publication of application:
**08.01.92 Bulletin  92/02**

(45) Publication of the grant of the patent:
**27.07.94 Bulletin  94/30**

(84) Designated Contracting States:
**AT BE CH DE DK ES FR GB IT LI LU NL SE**

(56) References cited:
**EP-A- 0 142 192**
**EP-A- 0 210 412**
**WO-A-84/00540**
**WO-A-88/03151**
**FR-A- 2 288 105**

**Journal of Cellular Physiology, vol. 124, 1985,
Alan R. Liss, Inc., D.C. Rideout et al .: "Am-
phiphilic cationic peptides mediate cell ad-
hesion to plastic surfaces", pages 365-371**

(73) Proprietor: **LA JOLLA CANCER RESEARCH
FOUNDATION**
**10901 North Torrey Pines Road**
**La Jolla, CA 92037(US)**

(72) Inventor: **PIERSCHBACHER, Michael, D.**
**766 Amiford Drive**
**San Diego, CA 92107(US)**
Inventor: **HONSIK, Cyril, J.**
**7539 High Avenue**
**La Jolla, CA 92037(US)**
Inventor: **Dreisbach, Lisa, B.**
**2513 Ocean Cove Drive**
**Cardiff, CA 92007(US)**

(74) Representative: **UEXKÜLL & STOLBERG Paten-
tanwälte**
**Beselerstrasse 4**
**D-22607 Hamburg (DE)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

EP 0 464 140 B1

**The Journal of Biological Chemistry, vol. 260, no. 7, 10 April 1985 , The Amercian Society of Biological Chemists, Inc., (US), M. Ginsberg et al.: "Inhibition of fibronectin binding to platelets by proteolytics fragments and synthetic peptides which support fibroblast adhension", pages 3931-3936**

**Description**

This invention relates to active adhesion peptides comprising an RGD-containing cell attachment promoting binding site and a hydrophobic domain to facilitate their attachment to a solid substrate.

A variety of assays and purification techniques require that a ligand, such as a peptide, be immobilized on a solid support. Generally, two methods have been used to accomplish such immobilization. The peptide may be applied to the surface in solution, which is then evaporated off, leaving the peptide dried to the surface. Such non-specific attachment is inefficient for small peptides and applicable only to methods which do not require a large concentration of immobilized peptide, as much will be resolubilized subsequently in the presence of solution. Moreover, because the attachment is non-specific, peptides will be attached in random and variant orientations. Where presentation of a particular active site is critical, such variance can further reduce the specificity of the bound peptide.

In the more common two-step chemical coupling process, the solid surface is first passively coated with a large protein, such as an immunoglobulin or bovine serum albumin. A hetero-bifunctional cross-linking agent, such as N-Succinimidyl 3-(2-pyridyldithio) propionate or glutaraldehyde, is attached to the protein and used to capture the peptide from solution. Such a method, while time consuming, is currently used, for example, in cell culture procedures which require a high concentration of bound peptide.

It is now recognized that many cell-cell and cell-matrix interactions are mediated by an arginine-glycine-aspartic acid (Arg-Gly-Asp or RGD) amino acid domain which is common to various adhesion proteins. This binding site is recognized by receptors. Synthetic peptides containing such a domain may be used in a variety of applications, including the coating of tissue culture plates or prostheses and immobilization for the purpose of the purification of adhesion receptors on a column. Achieving the attachment of an RGD-containing peptide to the solid substrate entails the same problems as do other peptides. In addition, because the adhesion domain is small, it is important that the domain be accessible to ligands, as by using a spacing group to distance the tripeptide from the surface that is being coated. Moreover, where the coating is for in vivo use, such as with a prosthesis, it is important that the coats be non-immunogenic and non-cytotoxic.

There thus exists a need for a rapid and reproducible one step-process for attaching peptides, such as RGD containing peptides, to a solid surface. Ideally, such a method should be easy to perform and efficient. In addition, it should preferable result in appropriate presentation of critical epitopes, such as the RGD domain. The present invention satisfies these needs and provides related advantages as well.

SUMMARY OF THE INVENTION

The invention provides a method of attaching active RGD-containing adhesion peptides to a surface through a hydrophobic domain while retaining their activity, and peptides so attached. The hydrophobic domain can contain either hydrophobic amino acids, such as leucine, valine, isoleucine or phenylalanine, or fatty acids, such as, for example, myristic acid, palmitic acid, arachidic acid or other fatty acids or hydrophobic moieties. Additionally, spacers, such as amino acids, between the hydrophobic domain and the biologically active domain can improve the presentation of the biologically active site. Specific peptides of the invention include

$GRGDSPASSKG_4 SRL_6 RNH_2$;
$GRGDSPASSKS_3 RL_6 RNH_2$;

AND

$GRGDSPASSKSSKRL_6 RNH_2$.

Coupled to a solid surface, such peptides can be used to detect the presence of a ligand complementary to an RGD-containing adhesion site.

DETAILED DESCRIPTION OF THE INVENTION

The present invention relates to the use of hydrophobic attachment domains, such as hydrophobic amino acids or fatty acids, on peptides having an RGD-containing cell attachment promoting binding site to facilitate the attachment of the peptide to solid substrates. Such methods of specifically attaching synthetic peptides using hydrophobic domains are useful for localizing a broad range of synthetic peptides having

3

these RGD-containing adhesion sites to a variety of surfaces. The method, which bypasses the conventional two-step chemical coupling process, provides a rapid and reproducible method for presenting the RGD active site, for binding both with specific receptors as well as with specific cell types. Moreover, the coating can be relatively non-immunogenic and non-cytotoxic, particularly when naturally occurring amino acids and fatty acids are used.

RGD is known to comprise the binding site of various extracellular matrix proteins, such as fibronectin. RGD containing peptides coated on a substrate promote cell attachment. Alternatively, in soluble form, RGD containing peptides inhibit attachment or promote detachment of cells from a substrate. The arginine residue can be in the D- or L-configuration. See United States Patent Nos. 4,578,079; 4,616,517; and 4,792,525 and Serial No. 738,078, all of which are incorporated herein by reference.

An active adhesion peptide is constructed comprising an RGD-containing cell attachment promoting binding site and a hydrophobic attachment domain, preferably at the carboxyl terminus of the biologically active site of interest, wherein hydrophobicity is between those exhibited by the amino acid sequences $L_4$ and $L_6$, wherein said adhesion peptide will form a coating on solid surfaces when driven from solution in an aqueous environment.

The hydrophobic domain can comprise either multiple hydrophobic amino acid residues, such as leucine, valine, isoleucine or phenylalanine or fatty acids such as myristate $CH_3(CH_2)_{12}COO-$, palmitate $CH_3(CH_2)_{14}COO-$, arachidate $CH_3(CH_2)_{18}COO-$, etc. Other chemical moieties which are hydrophobic can also be utilized, including non-natural or non-biological amino acids and other organic or inorganic compounds. In one embodiment, the hydrophobic domain comprises multiple leucine residues, such as $L_6NH_2$ or $L_4NH_2$. Alternatively, amino acid sequences such as phenylalanine, isoleucine or valine may be used.

In order to have sufficient hydrophobicity to facilitate attachment, the hydrophobic attachment domain must have at least the hydrophobicity of the sequence $L_4$. As used herein, "hydrophobic attachment domain" refers to a domain having a hydrophobicity at least equivalent to the degree of hydrophobicity exhibited by the amino acid sequence $L_4$.

The addition of spacer sequences of about two to four amino acids between the RGD-containing cell attachment promoting binding site and the hydrophobic domain can improve presentation of the RGD-containing adhesion site. These spacers can be amino acids, for example glycine or serine. Preferably more than one residue is used, such as $S_2$, $S_3$ or $G_4$. In addition, other organic compounds such as sugars, or other carbon containing, non-hydrophobic moieties can be used.

Less specific cell attachment can be achieved using as the attachment site certain positively charged amino acids known to, i.e. capable of promote attachment, such as arginine, lysine, homoarginine or ornithine in combination with the hydrophobic attachment domain. For example, both $R_6GL_6NH_2$ and $RL_6RNH_2$ have been shown to have some cell attachment promoting activity.

When in solution, the peptide can be used to coat surfaces such as tissue culture apparatus, prosthetic implant devices, columns for cell or receptor isolation and surfaces for ELISA. The surfaces can be hydrophobic or hydrophilic. The hydrophobic domain will adhere to such surfaces spontaneously as it is driven from solution in an aqueous environment. Greater peptide coating can be achieved by drying the peptide solution down onto the surface. Appropriate surfaces include Millicell™-CM, Immobilon™, polystyrene, polycarbonate, Teflon (polytetrafluorethylene), silicone, polyurethane, polylactic acid, titanium, polyethylene, Gortex expanded polytetrafluorethylene, tooth dentine and cementum.

Where the peptide has cell adhesion promoting activity, the method provides an improved means of cell culture in serum free conditions. This method also has application for prosthetic devices where it is desirable to have cells attach to the implanted device. Additionally by reproducing a natural extracellular matrix binding site on the substrate, the production of cellular proteins requiring such a substrate can be increased.

Coupled to a surface, the peptides of the invention can be advantageously used to detect or isolate ligands complementary to an RGD-containing adhesion site. Such ligands can be of a wide variety of types. Using a hydrophobic attachment domain in conjunction with an RGD-binding site facilittes the detection and isolation of receptors which recognize the RGD site. The presence of the ligand bound to the RGD-containing adhesion site can be determined by a variety of methods, such as the use of a labelled anti-ligand antibody. Such formats and labels are well known to those skilled in the art.

The use of naturally occurring amino acids or fatty acids is preferable to other hydrophobic moieties when coating prosthetic devices to be used in humans because they are relatively non-immunogenic and non-toxic.

The following standard abbreviations are used herein to identify amino acid residues.

4

TABLE I

| Amino Acid | Three-letter Abbreviation | One-letter Symbol |
|---|---|---|
| Alanine | Ala | A |
| Arginine | Arg | R |
| D-Arginine | D-Arg | dR |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| nMe refers to an n-Methyl group. | | |

All amino acids are in the L-configuration unless otherwise specified.

The following examples are intended to illustrate but do not limit the invention.

EXAMPLE I

PRODUCTION OF THE PEPTIDES

Peptides were synthesized using an automated peptide synthesizer (Model 430A; Applied Biosystems, Foster City, CA), using the instructions provided by the manufacturer, and purified by reverse phase HPLC on a Biogel TSK SP-5-PW cation exchange column (Bio-Rad Laboratories, Richmond, CA).

EXAMPLE II

ATTACHMENT OF PEPTIDES

The following peptides were tested for their ability to spontaneously adherer to the plastic wells and subsequently promote cell attachment: $XG(R)GDSPASSKL_2NH_2$, $XG(R)GDSPASSKL_4NH_2$, $XG(R)$-$GDSPASSKL_6NH_2$, $XG(R)GDSPASSK_6$, $XG(R)GDSPASSE_6$, where X is $NH_2$, nMe, acetyl or other amino acid. The peptides were solubilized in 70% EtOH at a starting concentration of 1 mg/ml. 200 $\mu$l was then added in duplicate to the first two wells of ninety-six well polystyrene microtiter plates that had not been treated for tissue culture. The remaining wells contained 100 $\mu$l of 70% EtOH. Taking 100 $\mu$l from the 1 mg/ml solution in the first two wells, a serial dilution was performed out to 0.15 $\mu$g/well for each peptide. The plates were allowed to dry overnight at 37°C in the presence of a desiccator.

The following day the peptide coated plates were used in a standard adhesion assay as described in Example III, using MG-63 cells, an osteosarcoma cell line available from American Type Tissue Culture.

EXAMPLE III

## CELL ADHESION ASSAY

The plates were washed 1X using PBS, (150 mM NaCl/10 mM sodium phosphate, pH 7.4). The plates were incubated with DMEM (0.1 ml/well) containing bovine serum albumin (BSA 2.5 mg/ml; Sigma Chemical Co., St. Louis, MO) for one hour. Two x $10^5$ cells/ml (MG-63) were suspended in DMEM, and BSA (2.5 mg/ml). 100 $\mu$l of cell suspension was added per well and the plates were incubated at 37°C in 7.5% $CO_2$/92.5% air for one hour. Afterwards, the plate was washed once in PBS. Attached cells were then fixed with 3% paraformaldehyde and stained overnight with 1% toluidine blue in 10% formaldehyde. The following day, the excess stain was removed and the plates were washed with $H_2O$; 100 $\mu$l of a 1% sodium dodecyl sulfate (SDS) solution was added to each well. The level of cell attachment was then measured, using a kinetic microplate reader (Molecular Devices,) by assessing the optical density at 630 nm.

The results are presented in Table II. XG(R)GDSPASSKL$_6$NH$_2$ promoted significant cell attachment out to a 3 $\mu$g/well. XG(R)GDSPASSKL$_4$NH$_2$ promoted marginal cell attachment only at the highest concentration of 100 $\mu$g/well. The other peptides nMeG(dR)GDSPASSKL$_2$NH$_2$, nMeG(dR)GDSPASSK$_6$, nMeG(dR)-GDSPASSE$_6$ did not promote significant cell attachment (Table II).

### TABLE II

| $\mu$g/well | 100 | 50 | 25 | 12 | 6 | 3 | 1.5 |
|---|---|---|---|---|---|---|---|
| Peptide | | | | | | | |
| $K_6$ | 3 | 2 | 2 | 2 | 2 | 3 | 2 |
| $E_6$ | 8 | 5 | 3 | 3 | 2 | 2 | 2 |
| $L_2$ | 6 | 6 | 3 | 2 | 1 | 1 | 1 |
| $L_4$ | 44 | 12 | 16 | 10 | 2 | 1 | 1 |
| $L_6$ | 100 | 76 | 60 | 60 | 60 | 5 | 3 |

The values represent percentage of maximum binding.

EXAMPLE IV

## PHENYLALANINE AS A HYDROPHOBIC CARRIER

The hydrophobic attachment of RGD containing peptides can be achieved with other hydrophobic amino acids. The following peptide, nMeG(dR)GDSPASSKRF$_4$RNH$_2$ was solubilized in 70% EtOH and used in an adhesion assay as described in Example III. This experiment also demonstrated the increase in cell binding by using an $S_3$ spacer on an RF$_6$RNH$_2$ tail. GRGESPS$_8$L$_6$NH$_2$ was used as a negative control, in that RGE is known not to have cell binding activity. The results are presented in Table III.

### TABLE III

| PHENYLALANINE AS A HYDROPHOBIC DOMAIN | | | | | | | |
|---|---|---|---|---|---|---|---|
| $\mu$g/well | 100 | 50 | 25 | 12 | 6 | 3 | 1.5 |
| nMeGRGDSPASSKS$_3$RF$_6$RNH$_2$ | 69 | 69 | 60 | 66 | 65 | 70 | 70 |
| nMeGRGDSPASSKRF$_6$RNH$_2$ | 70 | 75 | 56 | 52 | 57 | 54 | 50 |
| nMeGRGDSPASSKL$_6$NH$_2$ | 80 | 78 | 58 | 66 | 60 | 56 | 47 |
| nMeGRGDSPASSKRL$_5$RNH$_2$ | 72 | 68 | 58 | 66 | 60 | 44 | 34 |
| nMeGRGDSPASSKRF$_4$RNH$_2$ | 70 | 73 | 60 | 59 | 56 | 50 | 34 |
| GRGESPS$_8$L$_6$NH$_2$ | 27 | 17 | 16 | 10 | 16 | 8 | 3 |

The values represent percentage of maximum binding.

EXAMPLE V

MYRISTIC ACID AS A HYDROPHOBIC CARRIER

Fatty acids can also be used to promote the hydrophobic attachment of RGD containing peptides. Myristic acid was attached to the peptide, nMeG(dR)GDSPASSK via the epsilon amino group of the lysine. Cell attachment activity was determined in Examples II and III.

TABLE IV

| $\mu$g/well | 100 | 50 | 25 | 12 | 6 |
|---|---|---|---|---|---|
| nMeG(dR)GDSPASSK-Myristic acid | 33 | 30 | 50 | 75 | 45 |
| nMeGRGDSPASSKL$_6$-NH$_2$ | 100 | 100 | 100 | 50 | 35 |
| The values represent percentage of maximum binding. | | | | | |

EXAMPLE VI

Cell attachment can also be promoted using positive charge alone on a hydrophobic tail, such as the peptide R$_6$GL$_6$. The plastic wells were coated with peptide as in Example II and used in the assay of Example III.

TABLE V

| $\mu$g/well | 300 | 150 | 75 | 25 | 12 | 6 |
|---|---|---|---|---|---|---|
| R$_6$GL$_6$ | 70 | 60 | 50 | 50 | 50 | 43 |
| nMeG(dR)GDSPASSKL$_6$-NH$_2$ | 100 | 85 | 65 | 50 | 50 | 45 |
| The values represent percentage of maximum binding. | | | | | | |

EXAMPLE VII

The addition of amino acid spacers between the hydrophobic domain in the RGD binding site enhances the presentation of the RGD peptide for cell binding. The plastic wells were coated with peptide as in Example II, for four hours.

TABLE VI

| Peptides | $\mu$g/well | 10 | 5 | 2.5 | 1.25 | 6 |
|---|---|---|---|---|---|---|
| nMeG(dR)GDSPASSKS$_4$RL$_6$RNH$_2$ | | 57 | 76 | 100 | 92 | 76 |
| nMeG(dR)GDSPASSKS$_2$RL$_6$RNH$_2$ | | 34 | 53 | 57 | 46 | 38 |
| nMeG(dR)GDSPASSKL$_6$NH$_2$ | | – | 65 | 65 | 12 | 9 |

The values represent percentage of maximum binding.

EP 0 464 140 B1

EXAMPLE VIII

Phosphatidylcholine liposomes incorporating cell surface receptors were prepared essentially by the method of Mimms et al., Biochemistry 20:833 (1981), which is incorporated herein by reference. The vitronectin receptor and IIb/IIIa were isolated from placenta according to the method of Pytela, Meth. Enzym., 144:475 (1987), which is incorporated herein by reference. Briefly, 50 $\mu$g of egg yolk phosphatidylcholine (Sigma, St. Louis, MO) and [3H]phosphatidylcholine (1-2 $\mu$Ci, New England Nuclear, Boston, MA) were added to 1 ml of the appropriate receptor fractions (concentration of receptor, 1-20 $\mu$g/ml). This solution was then dialyzed against detergent-free PBS containing 1 mM $CaCl_2$ and 1 mM $MgCl_2$ for 24 hours at 4°C. This procedure results in the formation of liposomes with an average diameter of approximately 200 nm, as judged by electron microscopy. Ninety six well microtiter plates (Titertek, Flow Laboratories, Inc., McLean, VA) were coated at room temperature overnight with either the hydrophobic peptide (AC)GRGDSPASSKG$_4$SRL$_6$RNH$_2$, extracellular matrix proteins (vitronectin (VN), fibrinogen (FG)), or bovine serum albumin (BSA). The plates were blocked with 10% non-fat milk in PBS for 6 hours at 4°C and washed with cold Tris buffered saline (TBS). The plates were incubated with the receptor-containing liposomes for 32 hours at 4°C. After washing with cold TBS, bound liposomes were solubilized with 1% SDS and the released radioactivity was measured in a beta-counter (Beckman, Model LS5000CE).

(AC)GRGDSPASSKG$_4$SRL$_6$RNH$_2$ (PepTite-2000™ Telios Pharmaceuticals, Inc., La Jolla, CA) was shown to be a viable substrate for both the vitronectin receptor (Table VII) and the IIb/IIIa receptor (Table VIII) in liposome receptor studies. When the RGD sequence was omitted from the peptide (Pept-), resulting in the sequence GSPASSKRL$_6$RNH$_2$, vitronectin and IIb/IIIa integrin receptor binding was lost.

Table VII

| Vitronectin Receptor Liposome Assay | | |
|---|---|---|
| | 12.5 $\mu$g/ml Substrate | 25 $\mu$g/ml Substrate |
| Vitronectin | 1782 | 1808 |
| PepTite-2000™ | 1788 | 1921 |
| Pept- | 287 | 513 |
| BSA | - | 450 |
| The values represent cpm | | |

Table VIII

| IIb/IIIa Receptor Liposome Assay | | |
|---|---|---|
| | 12.5 $\mu$g/ml Substrate | 25 $\mu$g/ml Substrate |
| Fibrinogen | 3636 | 3958 |
| PepTite-2000™ | 1888 | 2307 |
| Pept- | 14 | 120 |
| BSA | - | 40 |

Although the invention has been described with reference to the presently-preferred embodiment, it should be understood that various modifications can be made by those skilled in the art without departing from the invention. Accordingly, the invention is limited only by the following claims.

Claims
Claims for the following Contracting States : AT, BE, CH, DE, DK, FR, GB, IT, LI, LU, NL, SE

1. An active adhesion peptide comprising an RGD-containing cell attachment promoting binding site and a hydrophobic attachment domain having hydrophobicity between those exhibited by the amino acid sequences L$_4$ and L$_6$, wherein said adhesion peptide will form a coating on solid surfaces when driven from solution in an aqueous environment.

8

2. The peptide of claim 1 wherein said amino acids of the hydrophobic domain are selected from the group consisting of leucine, isoleucine, phenylalanine and valine.

3. The active adhesion peptide of claim 1 or 2, further comprising a spacer sequence of about two to four amino acids between said cell attachment promoting binding site and said hydrophobic domain.

4. The peptide of claim 3 wherein said spacer comprises residues of serine or glycine.

5. A method for attaching active RGD-containing adhesion peptides according to any of the claims 1 to 4 to a surface while retaining their activity, comprising the steps of:
   a) providing on said peptide a domain having hydrophobicity between that expressed by the amino acid sequences $L_4$ and $L_6$; and
   b) allowing said peptide to attach to a surface.

6. The method of claim 5 wherein said surface is composed of polystyrene, polycarbonate, teflon, silicone, polyurethane, polylactic acid, titanium, polyethylene, expanded polytetrafluoroethylene, polyglycolic acid, tooth dentine or cementum.

7. A composition of matter comprising an active RGD-containing adhesion peptide having a hydrophobic domain having hydrophobicity between those exhibited by the amino acid sequences $L_4$ and $L_6$ adsorbed to a surface.

8. The composition of claim 7 wherein said surface is selected from the group consisting of protheses, dental implants and tissue culture apparatus.

9. A method for detecting the presence in a sample of a ligand complementary to an RGD-containing adhesion site, comprising:
   a) contacting said sample with the composition of claim 7 so as to allow binding between any said ligand in said sample and said RGD-containing adhesion site; and
   b) determining the presence of said complementary ligand bound to said RGD-containing adhesion site.

10. A method of promoting the attachment of cells to a surface comprising providing the composition of matter of claim 7 and allowing cells to attach thereto.

11. The method of claim 10 wherein said surface is selected from the group consisting of protheses, dental implants and tissue culture apparatus.

12. The method of claim 10 wherein said surface is composed of polystyrene, polycarbonate, teflon, silicone, polyurethane, polylactic acid, titanium, polyethylene, expanded polytetrafluoroethylene, polyglycolic acid, tooth dentine or cementum.

13. A peptide having the amino acid sequence

X-GRGDSPASSK$G_4$SR$L_6$RNH$_2$,

wherein X is NH$_2$, nME, acetyl or other amino acid.

14. A peptide having the amino acid sequence

X-GRGDSPASSK$S_3$RL$_6$RNH$_2$,

wherein X is NH$_2$, nMe, acetyl or other amino acid.

15. A peptide having the amino acid sequence

X-GRGDSPASSKSSKR$L_6$RNH$_2$,

wherein X is $NH_2$, nMe, acetyl or other amino acid.

**Claims for the following Contracting State : ES**

1. A method for the production of an active adhesion peptide which will form a coating on solid surfaces when driven from solution in an aqueous environment, characterized in that the peptide comprises an RGD-containing cell attachment promoting binding site and a hydrophobic attachment domain having hydrophobicity between those exhibited by the amino acid sequences $L_4$ and $L_6$.

2. The method of claim 1, wherein said amino acids of the hydrophobic domain are selected from the group consisting of leucine, isoleucine, phenylalanine and valine.

3. The method of claim 1 or 2, wherein the active adhesion peptide further comprises a spacer sequence of about 2 or 4 amino acids between said cell attachment promoting binding site and said hydrophobic domain.

4. The method of claim 3, wherein said spacer comprises residues of serine or glycine.

5. A method for attaching active RGD-containing adhesion peptides according to any of the claims 1 to 4 to a surface while retaining their activity, comprising the steps of:
   a) providing on said peptide a domain having hydrophobicity between that expressed by the amino acid sequences $L_4$ and $L_6$; and
   b) allowing said peptide to attach to a surface.

6. The method of claim 5 wherein said surface is composed of polystyrene, polycarbonate, teflon, silicone, polyurethane, polylactic acid, titanium, polyethylene, expanded polytetrafluoroethylene, polyglycolic aid, tooth dentine or cementum.

7. A composition of matter comprising an active RGD-containing adhesion peptide having a hydrophobic domain having hydrophobicity between those exhibited by the amino acid sequences $L_4$ and $L_6$ adsorbed to a surface.

8. The composition of claim 7, wherein said surface is selected from the group consisting of protheses, dental implants and tissue culture apparatus.

9. A method for detecting the presence in a sample of a ligand complementary to an RGD-containing adhesion site, comprising:
   a) contacting said sample with the composition of claim 7 so as to allow binding between any said ligand in said sample and said RGD-containing adhesion site; and
   b) determining the presence of said complementary ligand bound to said RGD-containing adhesion site.

10. A method of promoting the attachment of cells to a surface comprising providing the composition of matter of claim 7 and allowing cells to attach thereto.

11. The method of claim 10 wherein said surface is selected from the group consisting of protheses, dental implants and tissue culture apparatus.

12. The method of claim 10 wherein said surface is composed of polystyrene, polycarbonate, teflon, silicone, polyurethane, polylactic acid, titanium, polyethylene, expanded polytetrafluoroethylene, polyglycolic acid, tooth dentine or cementum.

13. The method of claims 1 to 4 wherein the peptide has the amino acid sequence

X-GRGDSPASSKG$_4$SRL$_6$RNH$_2$,

wherein X is $NH_2$, nMe, acetyl or other amino acid.

**14.** The method of claims 1 to 4 wherein the peptide has the amino acid sequence

X-GRGDSPASSKS$_3$RL$_6$RNH$_2$,

wherein X is NH$_2$, nMe, acetyl or other amino acid.

**15.** The method of claims 1 to 4 wherein the peptide has the amino acid sequence

X-GRGDSPASSKSSKRL$_6$RNH$_2$,

wherein X is NH$_2$, nMe, acetyl or other amino acid.

**Patentansprüche**
**Patentansprüche für folgende Vertragsstaaten : AT, BE, CH, DE, DK, FR, GB, IT, LI, LU, NL, SE**

**1.** Aktives Adhäsionspeptid, umfassend eine RGD enthaltende, die Zellanheftung fördernde Bindungsstelle und eine hydrophobe Anheftungsdomäne mit einer Hydrophobizität, die zwischen denjenigen liegt, die durch die Aminosäuresequenzen L$_4$ und L$_6$ ausgedrückt werden, wobei das Adhäsionspeptid eine Beschichtung fester Oberflächen bildet, wenn es aus einer Lösung in eine wäßrige Umgebung überführt wird.

**2.** Peptid nach Anspruch 1, bei dem die Aminosäuren der hydrophoben Domäne ausgewählt sind aus der Gruppe bestehend aus Leucin, Isoleucin, Phenylalanin und Valin.

**3.** Aktives Adhäsionspeptid nach Anspruch 1 oder 2, ferner umfassend eine Spacer-Sequenz von etwa 2 bis 4 Aminosäuren zwischen der die Zellanheftung fördernden Bindungsstelle und der hydrophoben Domäne.

**4.** Peptid nach Anspruch 3, bei dem der Spacer Serin- oder Glycinreste umfaßt.

**5.** Verfahren zur Anheftung aktiver, RGD enthaltender Adhäsionspeptide gemäß einem der Ansprüche 1 bis 4 an eine Oberfläche unter Erhalt ihrer Aktivität, umfassend die Schritte:
a) Schaffung einer Domäne auf dem Peptid mit einer Hydrophobizität, die zwischen denjenigen liegt, die durch die Aminosäuresequenzen L$_4$ und L$_6$ ausgedrückt werden, und
b) Ermöglichung der Anheftung des Peptids an eine Oberfläche.

**6.** Verfahren nach Anspruch 5, bei dem die Oberfläche zusammengesetzt ist aus Polystyrol, Polycarbonat, Teflon, Silikon, Polyurethan, Polymilchsäure, Titan, Polyethylen, expandiertem Polytetrafluorethylen, Polyglykolsäure, Zahndeutin oder -zement.

**7.** Stoffzusammensetzung, umfassend ein aktives, RGD enthaltendes Adhäsionspeptid mit einer hydrophoben Domäne mit einer Hydrophobizität, die zwischen denjenigen liegt, die durch die Aminosäuresequenzen L$_4$ und L$_6$ ausgedrückt werden, adsorbiert an eine Oberfläche.

**8.** Zusammensetzung nach Anspruch 7, bei der die Oberfläche ausgewählt ist aus der Gruppe bestehend aus Prothesen, Zahnimplantaten und Vorrichtungen für die Gewebekultur.

**9.** Verfahren zum Nachweis des Vorhandenseins eines gegenüber einer RGD enthaltenden Adhäsionsstelle komplementären Liganden in einer Probe, umfassend:
a) Kontaktieren der Probe mit der Zusammensetzung gemäß Anspruch 7, um eine Bindung zwischen irgendeinem in der Probe vorhandenen Liganden und der RGD enthaltenden Adhäsionsstelle zu erlauben, und
b) Bestimmung des Vorhandenseins des an die RGD enthaltende Adhäsionsstelle gebundenen komplementären Liganden.

**10.** Verfahren zur Förderung der Anheftung von Zellen an eine Oberfläche, umfassend die Schaffung der Stoffzusammensetzung gemäß Anspruch 7, und Ermöglichung der Anheftung der Zellen daran.

**11.** Verfahren nach Anspruch 10, bei dem die Oberfläche ausgewählt wird aus der Gruppe bestehend aus Prothesen, Zahnimplantaten und Vorrichtungen für die Gewebekultur.

**12.** Verfahren nach Anspruch 10, bei dem die Oberfläche zusammengesetzt ist aus Polystyrol, Polycarbonat, Teflon, Silikon, Polyurethan, Polymilchsäure, Titan, Polyethylen, expandiertem Polytetrafluorethylen, Polyglykolsäure, Zahndeutin oder -zement.

**13.** Peptid mit der Aminosäuresequenz $X\text{-GRGDSPASSKG}_4\text{SRL}_6\text{RNH}_2$, in der X $NH_2$, nME, Acetyl oder eine andere Aminosäure ist.

**14.** Peptid mit der Aminosäuresequenz $X\text{-GRGDSPASSKS}_3\text{RL}_6\text{RNH}_2$, in der X $NH_2$, nME, Acetyl oder eine andere Aminosäure ist.

**15.** Peptid mit der Aminosäuresequenz $X\text{-GRGDSPASSKSSKRL}_6\text{RNH}_2$, in der X $NH_2$, nME, Acetyl oder eine andere Aminosäure ist.

**Patentansprüche für folgenden Vertragsstaat : ES**

**1.** Verfahren zur Herstellung eines aktiven Adhäsionspeptids, das auf festen Oberflächen eine Beschichtung ausbildet, wenn es aus einer Lösung in eine wäßrige Umgebung überführt wird, dadurch gekennzeichnet, daß das Peptid eine RGD enthaltende, die Zellanheftung fördernde Bindungsstelle und eine hydrophobe Anheftungsdomäne umfaßt mit einer Hydrophobizität, die zwischen denjenigen liegt, die durch die Aminosäuresequenzen $L_4$ und $L_6$ ausgedrückt werden.

**2.** Verfahren nach Anspruch 1, bei dem die Aminosäuren der hydrophoben Domäne ausgewählt werden aus der Gruppe bestehend aus Leucin, Isoleucin, Phenylalanin und Valin.

**3.** Verfahren nach Anspruch 1 oder 2, bei dem das aktive Adhäsionspeptid ferner eine Spacer-Sequenz von etwa 2 bis 4 Aminosäuren zwischen der die Zellanheftung fördernden Bindungsstelle und der hydrophoben Domäne umfaßt.

**4.** Verfahren nach Anspruch 3, bei dem der Spacer Serin- oder Glycinreste umfaßt.

**5.** Verfahren zur Anheftung aktiver, RGD enthaltender Adhäsionspeptide gemäß einem der Ansprüche 1 bis 4 an eine Oberfläche unter Erhalt ihrer Aktivität, umfassend die Schritte:
a) Schaffung einer Domäne auf dem Peptid mit einer Hydrophobizität, die zwischen denjenigen liegt, die durch die Aminosäuresequenzen $L_4$ und $L_6$ ausgedrückt werden, und
b) Ermöglichung der Anheftung des Peptids an eine Oberfläche.

**6.** Verfahren nach Anspruch 5, bei dem die Oberfläche zusammengesetzt ist aus Polystyrol, Polycarbonat, Teflon, Silikon, Polyurethan, Polymilchsäure, Titan, Polyethylen, expandiertem Polytetrafluorethylen, Polyglykolsäure, Zahndeutin oder -zement.

**7.** Stoffzusammensetzung, umfassend ein aktives, RGD enthaltendes Adhäsionspeptid mit einer hydrophoben Domäne mit einer Hydrophobizität, die zwischen denjenigen liegt, die durch die Aminosäuresequenzen $L_4$ und $L_6$ ausgedrückt werden, adsorbiert an eine Oberfläche.

**8.** Zusammensetzung nach Anspruch 7, bei der die Oberfläche ausgewählt ist aus der Gruppe bestehend aus Prothesen, Zahnimplantaten und Vorrichtungen für die Gewebekultur.

**9.** Verfahren zum Nachweis des Vorhandenseins eines gegenüber einer RGD enthaltenden Adhäsionsstelle komplementären Liganden in einer Probe, umfassend:
a) Kontaktieren der Probe mit der Zusammensetzung gemäß Anspruch 7, um eine Bindung zwischen irgendeinem in der Probe vorhandenen Liganden und der RGD enthaltenden Adhäsionsstelle zu erlauben, und
b) Bestimmung des Vorhandenseins des an die RGD enthaltende Adhäsionsstelle gebundenen komplementären Liganden.

**10.** Verfahren zur Förderung der Anheftung von Zellen an eine Oberfläche, umfassend die Schaffung der Stoffzusammensetzung gemäß Anspruch 7, und Ermöglichung der Anheftung der Zellen daran.

**11.** Verfahren nach Anspruch 10, bei dem die Oberfläche ausgewählt wird aus der Gruppe bestehend aus Prothesen, Zahnimplantaten und Vorrichtungen für die Gewebekultur.

**12.** Verfahren nach Anspruch 10, bei dem die Oberfläche zusammengesetzt ist aus Polystyrol, Polycarbonat, Teflon, Silikon, Polyurethan, Polymilchsäure, Titan, Polyethylen, expandiertem Polytetrafluorethylen, Polyglykolsäure, Zahndeutin oder -zement.

**13.** Verfahren nach den Ansprüchen 1 bis 4, bei dem das Peptid die Aminosäuresequenz X-GRGDSPASSKG$_4$SRL$_6$RNH$_2$ aufweist, in der XNH$_2$, nME, Acetyl oder eine andere Aminosäure ist.

**14.** Verfahren nach den Ansprüchen 1 bis 4, bei dem das Peptid die Aminosäuresequenz X-GRGDSPASSKS$_3$RL$_6$RNH$_2$ aufweist, in der X NH$_2$, nME, Acetyl oder eine andere Aminosäure ist.

**15.** Verfahren nach den Ansprüchen 1 bis 4, bei dem das Peptid die Aminosäuresequenz X-GRGDSPASSKSSKRL$_6$RNH$_2$ aufweist, in der X NH$_2$, nME, Acetyl oder eine andere Aminosäure ist.

## Revendications
**Revendications pour les Etats contractants suivants : AT, BE, CH, DE, DK, FR, GB, IT, LI, LU, NL, SE**

**1.** Peptide d'adhérence actif comprenant un site de liaison contenant la séquence RGD favorisant l'attachement des cellules et un domaine d'attachement dont l'hydrophobicité est comprise entre celle que présente les séquences d'acides aminés L$_4$ et L$_6$, où ledit peptide d'adhérence formera un revêtement sur des surfaces solides lorsqu'il sort de la solution dans un environnement aqueux.

**2.** Peptide selon la revendication 1 dans lequel lesdits acides aminés-du domaine hydrophobe sont choisis au sein d'un groupe constitué par la leucine, l'isoleucine, la phénylalanine et la valine.

**3.** Peptide d'adhérence actif selon la revendication 1 ou 2, comprenant en outre une séquence de deux à quatre acides aminés servant d'espaceur entre ledit site de liaison facilitant l'attachement des cellules et ledit domaine hydrophobe.

**4.** Peptide selon la revendication 3 dans lequel l'espaceur comprend un résidu sérine ou glycine.

**5.** Procédé d'attachement de peptides d'adhérence actifs contenant une séquence RGD selon l'une des revendications 1 à 4 sur une surface tout en conservant leur activité, comprenant les étapes consistant à:
a) munir ledit peptide d'un domaine possédant une hydrophobicité comprise entre celles que présentent les séquences d'acides aminés L$_4$ et L$_6$; et
b) laisser ledit peptide s'attacher à une surface.

**6.** Procédé selon la revendication 5, dans lequel ladite surface est composée de polystyrène, de polycarbonate, de Téflon, de silicone, de polyuréthane, d'acide polylactique, de titane, de polyéthylène, de polytétrafluoroéthylène expansé, d' acide polyglycolique, de dentine ou d'amalgame dentaire.

**7.** Composition d'un matériau comprenant un peptide d'adhérence actif contenant une séquence RGD possédant un domaine hydrophobe dont l'hydrophobicité se situe entre celle que présentent les séquences d'acides aminés L$_4$ et L$_6$ adsorbé sur une surface.

**8.** Composition de la revendication 7 dans laquelle ladite surface est sélectionnée dans un groupe comprenant des prothèses, des implants dentaires et des appareils de culture de tissu.

**9.** Procédé de détection dans un échantillon de la présence d'un ligand complémentaire à un site d'adhérence contenant une séquence RGD comprenant:
a) la mise en contact dudit échantillon avec la composition de la revendication 7 de manière à permettre la liaison entre l'un desdits ligands dans ledit échantillon et ledit site d'adhérence contenant une séquence RGD; et

b) la détermination de la présence dudit ligand complémentaire lié audit site d'adhérence contenant une séquence RGD.

10. Procédé destiné à faciliter l'attachement des cellules sur une surface comprenant l'utilisation de la composition du matériau de la revendication 7 et l'attachement des cellules à ce matériau.

11. Procédé de la revendication 10 dans lequel ladite surface est choisie au sein d'un groupe comprenant des prothèses, des implants dentaires et des appareils de culture de tissus.

12. Procédé de la revendication 10 dans lequel ladite surface est composée de polystyrène, de polycarbonate, de Téflon, de silicone, de polyuréthane, d'acide polylactique, de titane, de polyéthylène, de polytétrafluoroéthylène expansé, d'acide polyglycolique, de dentine ou d'amalgame dentaire.

13. Peptide possédant la séquence d'acides aminés :

$X\text{-}GRGDSPASSKG}_4\,SRL_6\,RNH_2$,

où X est un groupe $NH_2$, nMe, acétyle ou un autre acide aminé.

14. Peptide possédant la séquence d'acides aminés :

$X\text{-}GRGDSPASSKS}_3\,RL_6\,RNH_2$,

où X est un groupe $NH_2$, nMe, acétyle ou un autre acide aminé.

15. Peptide possédant la séquence d'acides aminés :

$X\text{-}GRGDSPASSKSSKRL}_6\,RNH_2$,

où X est un groupe $NH_2$, nMe, acétyle ou un autre acide aminé.

**Revendications pour l'Etat contractant suivant : ES**

1. Procédé de préparation d'un peptide d'adhérence actif qui formera un revêtement sur des surfaces solides lorsqu'il sort de la solution dans un environnement aqueux, caractérisé en ce que le peptide comprend un site de liaison contenant une séquence RGD favorisant l'attachement des cellules et un domaine d'attachement hydrophobe dont l'hydrophobicité se situe entre celle des séquences d'acides aminés $L_4$ et $L_6$.

2. Procédé la revendication 1 dans lequel lesdits acides aminés du domaine hydrophobe sont choisis au sein d'un groupe constitué par la leucine, l'isoleucine, la phénylalanine et la valine.

3. Procédé selon la revendication 1 ou 2, dans lequel le peptide d'adhérence actif comprend en outre une séquence de deux à quatre acides aminés servant d'espaceur entre ledit site de liaison facilitant l'attachement des cellules et ledit domaine hydrophobe.

4. Procédé selon la revendication 3 dans lequel l'espaceur comprend un résidu sérine ou glycine.

5. Procédé d'attachement de peptides d'adhérence actifs contenant une séquence RGD selon l'une des revendications 1 à 4 sur une surface tout en conservant leur activité, comprenant les étapes consistant à:

a) munir ledit peptide d'un domaine possédant une hydrophobicité comprise entre celles que présentent les séquences d'acides aminés $L_4$ et $L_6$ ; et

b) laisser ledit peptide s'attacher à une surface.

6. Procédé selon la revendication 5, dans lequel ladite surface est composée de polystyrène, de polycarbonate, de Téflon, de silicone, de polyuréthane, d'acide polylactique, de titane, de polyéthylène, de polytétrafluoroéthylène, d'acide polyglycolique, de dentine ou d'amalgame dentaire.

7. Composition d'un matériau comprenant un peptide d'adhérence actif contenant une séquence RGD possédant un domaine hydrophobe dont l'hydrophobicité se situe entre celle que présentent les séquences d'acides aminés $L_4$ et $L_6$ adsorbé sur une surface.

8. Composition de la revendication 7 dans laquelle ladite surface est sélectionnée dans un groupe comprenant des prothèses, des implants dentaires et des appareils de culture de tissu.

9. Procédé de détection dans un échantillon de la présence d'un ligand complémentaire à un site d'adhérence contenant une séquence RGD comprenant:
   a) la mise en contact dudit échantillon avec la composition de la revendication 7 de manière à permettre la liaison entre l'un desdits ligands dans ledit échantillon et ledit site d'adhérence contenant une séquence RGD; et
   b) la détermination de la présence dudit ligand complémentaire lié audit site d'adhérence contenant une séquence RGD.

10. Procédé destiné à faciliter l'attachement des cellules sur une surface comprenant l'utilisation de la composition du matériau de la revendication 7 et l'attachement des cellules à ce matériau.

11. Procédé de la revendication 10 dans lequel ladite surface est choisie au sein d'un groupe comprenant des prothèses, des implants dentaires et des appareils de culture de tissus.

12. Procédé de la revendication 10 dans lequel ladite surface est composée de polystyrène, de polycarbonate, de Téflon, de silicone, de polyuréthane, d'acide polylactique, de titane, de polyéthylène, de polytétrafluoroéthylène expansé, d' acide polyglycolique, de dentine ou d'amalgame dentaire.

13. Procédé des revendications 1 à 4 dans lequel le peptide possède la séquence d'acides aminés :

   $X-GRGDSPASSKG_4 SRL_6 RNH_2$,

   dans laquelle X est un groupe $NH_2$, nMe, acétyle ou un autre acide aminé.

14. Procédé des revendications 1 à 4 dans lequel le peptide possède la séquence d'acides aminés :

   $X-GRGDSPASSKS_3 RL_6 RNH_2$,

   dans laquelle X est un groupe $NH_2$, nMe, acétyle ou un autre acide aminé.

15. Procédé des revendications 1 à 4 dans lequel le peptide possède la séquence d'acides aminés :

   $X-GRGDSPASSKSSKRL_6 RNH_2$,

   dans laquelle X est un groupe $NH_2$, nMe, acétyle ou un autre acide aminé.